# EUROPEAN PATENT SPECIFICATION

(11) **EP 2 416 849 B1**
(45) Date of publication and mention of the grant of the patent: **20.03.2013**
(21) Application number: 10717740.4
(22) Date of filing: 07.04.2010
(51) Int. Cl.: A61N 1/40, A61N 2/02

(54) **SET OF DEVICES FOR INFLUENCING THE OPERATION OF CELLS**
VORRICHTUNGSSATZ ZUR BEEINFLUSSUNG DER FUNKTION VON ZELLEN
ENSEMBLE DE DISPOSITIFS DESTINÉS À INFLUENCER LE FONCTIONNEMENT CELLULAIRE

(30) Priority: 09.04.2009 HU 0900218
(43) Date of publication of application: 15.02.2012
(73) Proprietor: Farkasné Lázár, Ibolya, 3400 Mezokovesd (HU)
(72) Inventor: Farkasné Lázár, Ibolya, 3400 Mezokovesd (HU)
(74) Representative: Ronaszéki, Tibor
(86) International application number: PCT/HU2010/000037
(87) International publication number: WO 2010/116197

(56) References cited:
- WO-A2-2004/080531
- US-A- 5 935 054
- US-A1- 2006 224 215

## Description

The invention relates to a set of devices for influencing the operation of cells, which comprises a central unit and a treatment part-unit connected to the central unit, the central unit has a driving part-unit for producing electric signals, and the driving part-unit is connected to the treatment part-unit with the help of a signal forwarding cable, the central unit has an external signal receiving input, the external signal receiving input is connected to a source unit suitable for reading alterable data content through the information forwarding channel, the driving part-unit of the central unit is supplemented with a signal amplifier part-unit, and the signal amplifier part-unit is connected to the external signal receiving input, and the treatment part-unit is formed by an antenna made of a metal wire placed in an embedding body, where the antenna is fitted with a connection gate, and the connection gate is connected to the central unit via the signal forwarding cable.

The methods of curing the human body have changed and developed continuously over the time depending on the different geographical regions or the cultural status of the different communities. Recently solutions different from the so-called traditional therapeutic methods have become increasingly widespread. Some of these solutions are based on the vibrations of the cells forming the living organism. It is known that all living organisms generate special vibrations having a frequency characteristic of the given structure. If the frequency is known, it is possible to influence the operation of the given cell, to terminate the abnormal operation of the cell and restore appropriate operation by "putting in" the given frequency or even a series of frequencies.

For example, patent description no. WO 99/66986 relates to equipment operating on the basis of this principle. The document describes a device for the treatment of a part of the biological body with magnetic fields, which is based on that a part of the biological body is placed in an active zone containing equipment for creating the magnetic field and equipment for creating an alternating magnetic field, and the equipment is realised as equipment generating so-called spin resonances in the biological body to be treated. During the procedure realised with the device the repetition time of a core spin resonance series is determined, and on the basis of this a core spin resonance series is generated.

Publication document no. US 2006/0224215 is also known of, it describes a digital electromagnetic pulse generator, which has a central unit with a power source connected to it and a treatment part-unit made of metal coils. In the central unit there is a microcontroller, which is suitable for generating certain waveforms, which then get to the coils of the treatment part-unit through the digital-to-analogue converter and through low-frequency power amplifiers, where they create an alternating electromagnetic space exerting a positive effect on the treated person.

However, the disadvantage of the solution is that only a limited number of waveforms selected using the program buttons placed on the keyboard of the central unit can be generated, so the given device cannot be used optimally for treating any disease of any person.

Another disadvantage of this solution is that with the help of the program buttons only a person skilled in the art is able to set the waveforms and other physical parameters, which enable the successful treatment of persons suffering from certain diseases.

It must also be regarded as a disadvantage that during treatment, when using the program buttons for setting the combined frequencies, in the course of the transition between the two selected frequencies certain upper harmonics may be generated and may get into the treatment part-unit and from there through the electromagnetic space into the treated person, which vibrations may be harmful to the patient. During the treatment of certain diseases, in a given case, vibrations set with a four-figure accuracy are needed, and any deviation from the set value may result in problems during treatment. The given equipment does not provide appropriate information relating to setting these treatment frequencies with a great accuracy.

However, the disadvantage of the known solutions is that they are not able to enter all known and well-defined frequencies into the living organism at cell level, which could have a therapeutic, restoring effect on the operation of the given living organism.

A further disadvantage is that the equipment suitable for treatment can generate and forward only a few frequencies that can restore basic alterations or faulty operation, so with their help it is not possible to perform an extensive therapeutic and roboration activity.

Another disadvantage is that the known solutions require complicated setting, so in each case a trained therapist is needed for their use. In this way the accessibility of the treatment becomes very restricted.

With the solution according to the invention our aim was to eliminate the deficiencies of the known solutions and create a set of devices, which makes it possible to generate and forward all known frequencies and enter them into the living organism in an appropriately efficient way, and due to the fact that its operation does not require any special professional knowledge and has a simple construction, it is an efficient device accessible to a wide layer of people.

The invention is based on the recognition that if one or more frequencies suitable for solving a given problem are placed on an independent data carrier device in a way other than usual, and then from this device the vibration or vibrations of the desired frequency are forwarded to the cells of the living organism with the help of a novel treatment unit, using electronic part-units connected to each other in a way other than usual, then vibrations of optional very low frequencies can be entered into the living organism to be treated, without needing a highly qualified specialist to set, operate or control the equipment, and by this the task can be solved, that is the possibility of an efficient, simple and widely accessible treatment can be provided.

In accordance with the set aim the set of devices for influencing the operation of cells according to the invention - which comprises a central unit and a treatment part-unit connected to the central unit, the central unit has a driving part-unit for producing electric signals, where the driving part-unit is connected to the treatment part-unit with the help of a signal forwarding cable, the central unit has an external signal receiving input, the external signal receiving input is connected to a source unit suitable for reading alterable data content through the information forwarding channel, the driving part-unit of the central unit is supplemented with a signal amplifier part-unit, and the signal amplifier part-unit is connected to the external signal receiving input, and the treatment part-unit is formed by an antenna made of a metal wire placed in an embedding body, where the antenna is fitted with a connection gate, and the connection gate is connected to the central unit via the signal forwarding cable - is constructed in such a way that the source unit is formed by a reading and processing device, e.g. a DVD player, with a laser reader head suitable for accommodating an independent data carrier device, and signal shapes containing vibration characteristics suitable for influencing the operation of cells are stored on the data carrier device cooperating with the source unit, the driving part-unit contains a FET final amplifier and a square signal generator, and the treatment part-unit is formed by 2-15 m long metal wire with a diameter of 0.5-3.5 mm in the shape of a wave, placed in an embedding body.

A further feature of the set of devices according to the invention may be that the embedding body contains a first rectangular cover layer made of a plate of an electrically insulated material and a second rectangular cover layer made of a plate of an electrically insulated material, and the antenna is inserted and fixed between the first cover layer and the second cover layer.

In the case of another version of the set of devices the antenna is formed by a group of segments ' made of a metal wire and bent in the shape of a planar square.

In the case of an even different realisation of the invention the treatment part-unit has an antenna made of twisted copper wire.

In the case of a further possible realisation of the set of devices the central unit is supplemented with a control part-unit, where the control part-unit has a sound emitting body and/or a light emitting body. The sound emitting body is a loudspeaker, while the light emitting body is an LED.

The set of devices according to the invention has numerous favourable features. The most important one of these is that due to the device constructed in a way other than the known devices and due to the novel data carrier, all known frequencies suitable for the treatment of different problems can be produced, and even frequencies in the range of low vibration numbers can be entered into the living organism to be treated without distortion, which could not be realised so far with the known devices.

A significant advantage of the solution is that different groups of frequencies for the treatment of a problem or group of problems existing in given persons are produced separately. As a result of this all treated persons can receive the most efficient "customised" treatment without needing the presence of a highly qualified specialists during the treatment

An important advantage is that the treatment frequencies that can be recorded on the data carrier can be altered suiting individual demands and needs, even per period, and in this way the treatment can be maintained at an optional level practically continuously. The desired group of frequencies can be defined during the check-up visits, and by this less load is put on specialists while their efficiency is increased significantly, which also improves the general efficiency of medical treatment, health restoration or health preservation to a great extent.

A further advantage is that the operation and use of the set of devices according to the invention is simple, it can be acquired without any special professional knowledge. The equipment can be used anywhere, even at home.

It must also be regarded as an advantage that due to the novel structural construction the production cost of the set of devices according to the invention can be kept at a very favourable level, which increases the possibility of its propagation even more.

A significant advantage deriving from the above is that the treatments can be realised anywhere and at any time, so the therapy itself can be continued at home, in a relaxed environment, which is an essential circumstance from the aspect of recovery too. Magnetic resonance treatments can be spread much more widely than it is possible using the presently known costly and complicated equipment requiring great professional knowledge.

Another advantage worth mentioning is that the set of devices according to the invention is a therapeutic device suitable for people and animals too, and from the aspect of its price it can also be obtained and used by animal keepers. As a result of this the treatment and rehabilitation of people and animals can be solved at home.

Below the set of devices according to the invention is described in detail in connection with a construction example, on the basis of a drawing. In the drawing

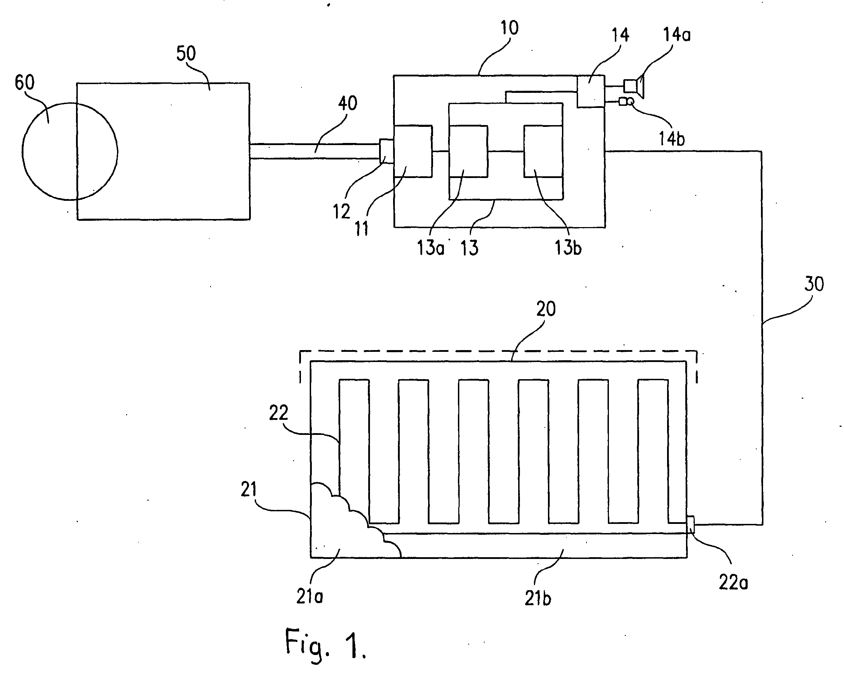

figure 1 is block diagram of a possible version of the set of devices according to the invention.

Figure 1 shows a construction of the set of devices according to the invention, in which the treatment part-unit 20 connected to the central unit 10 is formed by a 60 cm wide and 180 cm long mattress. It can be seen that the treatment part-unit 20 contains the antenna 22 placed inside the embedding body 21 containing the first cover layer 21 a and the second cover layer 21b, inserted between the first cover layer 21a and the second cover layer 21b. Here the antenna is formed from an 8,5 m long twisted copper wire 22 with a total diameter of 0.8 mm. The metal conductor forming the 22 consists of bent square-wave segments, where the individual segments are situated along the perimeter of a 12 cm wide and 34 cm long rectangle each. The antenna 22 constructed of such bent elements and built into the embedding body 21 is always present under the entire body surface of the person lying on the treatment part-unit 20.

A different treatment part-unit 20 with a different size and shape is also possible, but the main point in all treatment part-units 20 is that the antenna 22 made of a metal conductor, favourably copper wire, and bent to shape must be covered with an embedding body 21 made of an insulating material. In this way the antenna does not 22 get in contact with the body of the treated person, only the effect of the alternating magnetic field generated by the antenna is asserted on the body and in the cells of the person in contact with the treatment part-unit 20.

Apart from the mattress size suitable for treatment in a lying position, the treatment part-unit 20 can also be constructed as a belt to be attached on the waist - with the size of a kidney warmer belt - or even as a wristband. Obviously the greater the magnetic field extends, the more efficient the treatment will be.

The antenna 22 of the treatment part-unit 20 has a connection gate 22a placed on the embedding body 21. With the help of this connection gate 22a, via the signal forwarding cable 30 the antenna 22 is connected to the central unit 10. The central unit 10 - as shown in figure 1 - contains the driving part-unit 11, the signal amplifier part-unit 13 and the control part-unit 14. The signal amplifier part-unit 13 contains the final amplifier 13a and the square signal generator 13b. This signal amplifier part-unit 13 - which can also be constructed as a single integrated circuit element - creates the possibility of forwarding even very low vibrations at an accurate frequency value and with a clear signal shape towards the antenna 22, which then creates the necessary magnetic field in the narrow environment of the treatment part-unit 20.

The task of the control part-unit 14 is to make the operation of the central unit 10 perceivable with the help of the sound emitting body 14a and the light emitting body 14b. Favourably the sound emitting body 14a is a loudspeaker, which emits sound with a frequency passing through the central unit 10, while in the present case the light emitting body 14b is a multicolour LED. Another task of the light emitting body 14b is to show the operation and status of the central unit by changing the colour of the LED.

The central unit 10 has an external signal receiving input 12, to which the source unit 50 is connected with the help of the information forwarding channel 40. The source unit 50- in the case of this construction example - is a DVD reader with a laser reader head, which is suitable for reading the signals recorded on the data carrier device 60 and forwarding them to the central unit 10.

The data carrier device 60 is a CD, on which the image of the frequency or frequencies needed for the given treatment is recorded in a given length and number of periods. It must be pointed out here that the frequencies having an effect on different cells, tissues, organs or on the factors causing diseases in different ways do not form a part of the invention. Such data is accessible in public databases, for example it can be found on the internet too. However, the known devices can only produce and emit only a few frequencies, while the set of devices according to the invention, due to the data carrier device 60 that can be produced and varied independently, always contains the frequencies suiting the condition and diagnosis of the given treated person. This data can be entered into the central unit 10 with the help of the source unit 50.

Before using the set of devices according to the invention, the patient visits a doctor with his/her complaint, and the doctor - following the common protocol - sets up a diagnosis. When the disease to be treated has been diagnosed, the frequency or frequencies needed for destroying the pathogen or for restoring the appropriate operation of the given cells, tissues, organs can be selected, and 9 using any known data recording procedure - they can be recorded on the data carrier device 60.

The data carrier device 60 now containing the specific frequency characteristics is suitable for starting the treatment. At this point the person to be treated lies e.g.: on the first cover layer 21a of the embedding body of the mattress-like treatment part-unit 20. Then the source unit 50 is turned on, the data carrier device 60 is placed in the source unit 50, and the central unit 10 is started.

The source unit 50 reads the signals recorded on the data carrier device 60 and forwards them to the external signal receiving input 12 of the central unit 10. The signal arriving at the external signal receiving input 12 gets to the driving part-unit 11 of the central unit 10, from there to the final amplifier 13a of the signal amplifier part-unit 13, and then to the square signal generator 13b. After passing through the driving part-unit 11, the final amplifier 13a and the square signal generator 13b, the signal gets to the signal forwarding cable 30 in its final form, amplitude, frequency, band and repetition number, and then through the connection gate 22a to the antenna 22 of the treatment part-unit 20. The electric signal having given physical characteristics in the antenna 22 creates an alternating magnetic field around the embedding body 21 of the treatment part-unit 20, and also in the environment of the first cover layer 21 a of the embedding body 21.

The vibrations generated by the magnetic field created having a size and frequency alternating both in time and space have an effect on the cells situated in the environment of the first cover layer 21 a of the embedding body 21 of the treatment part-unit 20. These cells pass on the vibrations to the other cells, and so the vibrations at the frequency suitable for treatment are passed on to the target area, where the vibration exerts its effect. The effect mechanism itself is a process known in itself and discussed in the special literature. For this reason the operation of the solution is not described here, as the effect mechanism is not the subject of the invention.

On the one part the appropriate operation of the central unit 10 can be controlled on the basis of the light signals emitted by the light emitting body 14b of the control part-unit 14, and also by turning on the sound emitting body 14a of the control unit 14 and listening to the sound effects of the vibrations at different frequencies emitted towards the treatment part-unit 20.

After finishing the treatment the user turns off the central unit 10, takes out the data carrier device 60 containing the therapeutic vibrations prepared individually from the source unit 50, and turns off the source unit 50. The next person to be treated places his/her own data carrier device 60 in the source unit 50, which, depending on the data recorded on the data carrier device 60, generates vibrations at a frequency that may be completely different from the ones before, and forwards them via the central unit 10 to the antenna 22 of the treatment part-unit 20.

The set of devices according to the invention can be used favourably for supplementing or even replacing traditional therapies, and, depending on the frequency of the emitted vibrations, even for realising health preserving or improving treatments.

### List of references

- 10: central unit
11 driving part-unit
12 external signal receiving input
13 signal amplifier part-unit
13a final amplifier
13b square signal generator
14 control part-unit
14a sound emitting body
14b light emitting body
- 20: treatment part-unit
21 embedding body
21a first cover layer
21b second cover layer
22 antenna
22a connection gate
- 30: signal forwarding cable
- 40: information forwarding channel
- 50: source unit
- 60: data carrier device

## Claims

1. Set of devices for influencing the operation of cells forming a living organism, which comprises a central unit and a treatment part-unit connected to the central unit, the central unit has a driving part-unit for producing electric signals, where the driving part-unit is connected to the treatment part-unit with the help of a signal forwarding cable, the central unit has an external signal receiving input, the external signal receiving input is connected to a source unit suitable for reading alterable data content through the information forwarding channel, the driving part-unit of the central unit is supplemented with a signal amplifier part-unit, and the signal amplifier part-unit is connected to the external signal receiving input, and the treatment part-unit is formed by an antenna made of a metal wire placed in an embedding body, where the antenna is fitted with a connection gate, and the connection gate is connected to the central unit via the signal forwarding cable, wherein the source unit (50) is formed by a reading and processing device, e.g. a DVD player, with a laser reader head adapted to accommodate an independent data carrier device (60), and signal shapes containing vibration characteristics suitable for influencing the operation of said cells are stored on the data carrier device (60) cooperating with the source unit (50), wherein the source unit is adapted to read said signal shapes from the data carrier device and to forward the signal shapes to the central unit the driving part unit (13) contains a FET final amplifier (13a) and a square signal generator (13b), and the antenna (22) is formed by 2-15 m long metal wire with a diameter of 0.5-3.5 mm in the shape of a wave, placed in an embedding body (21).

2. Set of devices as in claim 1, **characterised by** that the embedding body (21) contains a first rectangular cover layer (21a) made of a plate of an electrically insulated material and a second rectangular cover layer (21b) made of a plate of an electrically insulated material, and the antenna (22) is inserted and fixed between the first cover layer (21a) and the second cover layer (21b).

3. Set of devices as in claim 1 or 2, **characterised by** that the antenna (22) is formed by a group of segments made of a metal wire and bent in the shape of a planar square wave.

4. Set of devices as in any of claims 1-3, **characterised by** that the treatment part-unit (20) has an antenna (22) made of twisted copper wire.

5. Set of devices as in any of claims 1-4, **characterised by** that the central unit (10) is supplemented with a control part-unit (14), where the control part-unit (14) has a sound emitting body (14a) and/or a light emitting body (14b).

6. Set of devices as in claim 5, **characterised by** that the sound emitting body (14a) is a loudspeaker.

7. Set of devices as in claim 5 or 6, **characterised by** that the light emitting body (14b) is an LED.

## Patentansprüche

1. Vorrichtungssatz zur Beeinflussung der Funktion von einen lebenden Organismus bildenden Zellen, der eine Zentraleinheit und eine mit der Zentraleinheit verbundene Behandlungsteileinheit umfasst, wobei die Zentraleinheit eine Antriebsteileinheit zur Erzeugung elektrischer Signale aufweist, wobei die Antriebsteileinheit mit Hilfe eines signalleitenden Kabels mit der Behandlungsteileinheit verbunden ist, wobei die Zentraleinheit einen externen Signalempfangseingang aufweist, wobei der externe Signalempfangseingang mit einer Quelleneinheit verbunden ist, die für ein Lesen eines veränderbaren Dateninhalts über den informationsleitenden Kanal geeignet ist, wobei die Antriebsteileinheit der Zentraleinheit mit einer Signalverstärkerteileinheit ergänzt ist, und wobei die Signalverstärkerteileinheit mit dem externen Signalempfangseingang verbunden ist, und wobei die Behandlungsteileinheit durch eine Antenne gebildet ist, die aus einem in einem Einbettungskörper angeordneten Metalldraht hergestellt ist, wobei die Antenne mit einem Verbindungsgatter versehen ist, und wobei das Verbindungsgatter über das signalleitende Kabel mit der Zentraleinheit verbunden ist, wobei die Quelleneinheit (50) von einer Lese- und Verarbeitungseinrichtung gebildet ist, z. B. einem DVD-Spieler, mit einem Laser-Lesekopf, der dafür ausgebildet ist, eine unabhängige Datenträgereinrichtung (60) aufzunehmen, und wobei Signalformen, die Vibrationscharakteristika enthalten, die für eine Beeinflussung der Funktion der Zellen geeignet sind, auf der mit der Quelleneinheit (50) zusammenwirkenden Datenträgereinrichtung (60) gespeichert sind, wobei die Quelleneinheit dafür ausgelegt ist, die Signalformen von der Datenträgereinrichtung zu lesen und die Signalformen an die Zentraleinheit weiterzuleiten, wobei die Antriebsteileinheit (13) einen FET-Endverstärker (13a) und einen Rechtecksignalgenerator (13b) aufweist, und wobei die Antenne (22) von einem 2 - 15 m langen Metalldraht mit einem Durchmesser von 0,5 - 3,5 mm in der Form einer Welle gebildet ist, der in einem Einbettungskörper (21) angeordnet ist.

2. Vorrichtungssatz nach Anspruch 1, **dadurch gekennzeichnet, dass** der Einbettungskörper (21) eine erste rechteckige Abdeckschicht (21 a) aufweist, die aus einer Platte aus einem elektrisch isolierten Material hergestellt ist, sowie eine zweite rechteckige Abdeckschicht (21 b), die aus einer Platte aus einem elektrisch isolierten Material hergestellt ist, und wobei die Antenne (22) zwischen der ersten Abdeckschicht (21a) und der zweiten Abdeckschicht (21b) eingesetzt und befestigt ist.

3. Vorrichtungssatz nach Anspruch 1 oder 2, **dadurch gekennzeichnet, dass** die Antenne (22) aus einer Gruppe von Segmenten gebildet ist, die aus einem Metalldraht hergestellt und in der Form einer ebenen Rechteckwelle gebogen sind.

4. Vorrichtungssatz nach einem der Ansprüche 1 bis 3, **dadurch gekennzeichnet, dass** die Behandlungsteileinheit (20) eine Antenne (22) aufweist, die aus einem verdrillten Kupferdraht hergestellt ist.

5. Vorrichtungssatz nach einem der Ansprüche 1 bis 4, **dadurch gekennzeichnet, dass** die Zentraleinheit (10) mit einer Steuerungsteileinheit (14) ergänzt ist, wobei die Steuerungsteileinheit (14) einen schallabstrahlenden Körper (14a) und/oder einen lichtabstrahlenden Körper (14b) aufweist.

6. Vorrichtungssatz nach Anspruch 5, **dadurch gekennzeichnet, dass** der schallabstrahlende Körper (14a) ein Lautsprecher ist.

7. Vorrichtungssatz nach Anspruch 5 oder 6, **dadurch gekennzeichnet, dass** der lichtabstrahlende Körper (14b) eine LED ist.

## Revendications

1. Ensemble de dispositifs destinés à influencer le fonctionnement de cellules constituant un organisme vivant, qui comprend une unité centrale et une unité de partie de traitement reliée à l'unité centrale, l'unité centrale comporte une unité de partie d'entraînement pour produire des signaux électriques, où l'unité de partie d'entraînement est reliée à l'unité de partie d'entraînement à l'aide d'un câble de transfert de signal, l'unité centrale a une entrée de réception de signal externe, l'entrée de réception de signal externe est reliée à une unité de source apte à lire des contenus de données modifiables par le biais du canal de transfert d'informations, l'unité de partie d'entraînement de l'unité centrale est complétée par une unité de partie d'amplificateur de signal, et l'unité de partie d'amplificateur de signal est reliée à l'entrée de réception de signal externe, et l'unité de partie de traitement est formée par une antenne constituée d'un fil métallique placé dans un corps d'encastrement, où l'antenne est pourvue d'une porte de liaison, et la porte de liaison est reliée à l'unité centrale par l'intermédiaire du câble de transfert de signal, dans lequel l'unité de source (50) est formée par un dispositif de lecture et de traitement, par exemple un lecteur DVD, avec une tête de lecteur laser apte à accueillir un dispositif porteur de données indépendant (60), et des formes de signal contenant des caractéristiques de vibrations aptes à influencer le fonctionnement desdites cellules sont stockées sur le dispositif porteur de données (60) en coopération avec l'unité de source (50), dans lequel l'unité de source est apte à lire lesdites formes de signal à partir du dispositif porteur de données et à transférer les formes de signal à l'unité centrale, l'unité de partie d'entraînement (13) comprend un amplificateur final à transistor à effet de champ, FET, (13a) et un générateur de signal carré (13b), et l'antenne (22) est formée par un fil métallique d'une longueur de 2 à 15 m et d'un diamètre de 0,5 à 3,5 mm sous la forme d'une onde, placée dans un corps d'encastrement (21).

2. Ensemble de dispositifs selon la revendication 1, **caractérisé en ce que** le corps d'encastrement (21) contient une première couche de recouvrement rectangulaire (21a) constituée d'une plaque d'un matériau isolé électriquement et une deuxième couche de recouvrement rectangulaire (21b) constituée d'une plaque d'un matériau isolé électriquement, et l'antenne (22) est insérée et fixée entre la première couche de recouvrement (21a) et la deuxième couche de recouvrement (21 b).

3. Ensemble de dispositifs selon la revendication 1 ou 2, **caractérisé en ce que** l'antenne (22) est formée par un groupe de segments constitués d'un fil métallique et pliés sous la forme d'une onde carrée plane.

4. Ensemble de dispositifs selon l'une quelconque des revendications 1 à 3, **caractérisé en ce que** l'unité de partie de traitement (20) comporte une antenne (22) constituée d'un fil de cuivre torsadé.

5. Ensemble de dispositifs selon l'une quelconque des revendications 1 à 4, **caractérisé en ce que** l'unité centrale (10) est complétée par une unité de partie de commande (14), où l'unité de partie de commande (14) comporte un corps d'émission de son (14a) et/ou un corps d'émission de lumière (14b).

6. Ensemble de dispositifs selon la revendication 5, **caractérisé en ce que** le corps d'émission de son (14a) est un haut-parleur.

7. Ensemble de dispositifs selon la revendication 5 ou 6, **caractérisé en ce que** le corps d'émission de lumière (14b) est une diode électroluminescente, DEL.
